# EUROPEAN PATENT SPECIFICATION

(11) **EP 0 232 165 B1**
(45) Date of publication and mention of the grant of the patent: **27.04.1994**
(21) Application number: 87300950.0
(22) Date of filing: 03.02.1987
(51) Int. Cl.: G01N 33/571, G01N 33/563, G01N 33/573, G01N 33/569

(54) **Diagnostic method for gonorrhea by assay of IgA1 fragments**
Diagnostisches Verfahren für Gonorrhoe durch Proben von IgA1-Fragmenten
Procédé diagnostique pour gonorrhée par l'essai des fragments de IgA1

(30) Priority: 05.02.1986 US 826227
(43) Date of publication of application: 12.08.1987
(73) Proprietor: IMMUNOGON ASSOCIATES, Great Neck New York (US)
(72) Inventor: Blake, Milan, New York, NY (US)
(74) Representative: Lawrence, Peter Robin Broughton

(56) References cited:
- EP-A- 0 236 605
- US-A- 4 245 038
- US-A- 4 248 964
- US-A- 4 525 452
- US-A- 4 582 699
- CHEMICAL ABSTRACTS, vol. 98, no. 17, 25 April 1983, Columbus, OH (US); J.V. GILBERT et al., p. 307, no. 140229e#
- CHEMICAL ABSTRACTS, vol. 104, no. 15, 18 April 1986, Columbus, OH (US); M.A. MULKS et al., p. 370, no. 126240n#

## Description

This invention concerns immunoassay of the fragments produced by the enzyme immunoglobulin A protease (IgAP) in the reaction with its substrate immunoglobulin A, sub-class 1 (IgA1). Bacteria which secrete IgAP may be detected by this immunoassay. The assay is especially useful in the detection of Neisseria gonorrhea and the diagnosis of gonorrhea.

Immunoglobin A protease (IgAP) has been the subject of intensive study since its discovery in the human body. IgAP is secreted by pathogenic bacteria and has been implicated in the diseases caused by these bacteria, namely gonorrhea, meningitis and influenza. The substrate of IgAP is immunoglobulin A, sub-class one (IgA1). IgA1 is naturally secreted in the human body by urogenital membranes as part of the natural immunity of mucous membranes. The role of IgAP in the pathogenicity of bacteria is not clear, however it is speculated that it is a defense mechanism of these bacteria which secrete IgAP.

The study of IgAP and its reaction with IgA1 would be aided by a rapid, simple assay for the enzyme. Current methods are cumbersome and time-consuming. Immunoelectrophoresis is a sophisticated technique which separates reaction products according to size and charge and then identifies them with anti-sera (Plaut, et al. (1974) Adv. Exp. Med. Biol., 245-249; Male, C.J. (1979) Inf. Immun., 26: 245-261). SDS-PAGE analysis of 125I-labelled IgA1 is a sensitive technique, but it is also time-consuming. (Blake, et al. (1979) J. Infect. Dis., 139: 89-92; Blake and Swanson (1978) Inf. Immun., 22: 350-358) Lindahl reports an assay utilising IgA-binding protein from Group A streptococci which is useful under laboratory conditions but this method is also limited by time and labour requirements (Lindahl, L. (1981) J. Clin. Microbiol., 13: 991-993).

In J. Immunol. Methods (1983) 57, 247-251 Gilbert et al describe the detection of fragments of IgA1 produced on cleavage by IgAP by an in vitro reaction of labelled IgA1 followed by separation and detection by auto radiography.

Accordingly, a rapid, simple assay of IgAP has been sought. A simple assay would allow detection of bacteria which secrete the enzyme and make possible early diagnosis of diseases caused by these bacteria.

In US-A-4,248,964 the identification of 1,2-propanedioldehydrogenase is achieved by the use of antibodies specific for the enzyme itself.

According to the present invention there is provided a new method for detection of IgA1, IgAP and/or bacteria secreting IgAP in a sample, wherein the fragments of IgA1 formed in vivo in a first step of enzymatic digestion of IgA1 by IgAP are subject to immunoassay of said fragments with monoclonal antibody capable of reacting specifically with neo-epitope produced by said enzymatic digestion, but incapable of reacting with intact IgA1.

The present invention sets forth a method for detecting fragments of immunoglobulin A, sub-class 1 (IgA1) produced by the enzymatic digestion of IgA1 by an immunoglobulin A-protease (IgAp). The method comprises immunoassay of the fragments with antibody which is capable of reacting with the fragments, but not with the undigested IgAp). The antibody used in the present assay is monoclonal antibody which is capable of reacting specifically with neo-epitopes found on the fragments of IgA1 produced after digestion of IgA1 with IgAP. These neo-epitopes are not found on intact IgA1.

Immunoassays involving the antibody comprise enzyme-linked immunoassay (ELISA), radioimmunoassay, sandwich assay, enzyme-inhibition immunoassay (EIIA) or any of the methods well known in the art. Enzyme-inhibition immunoassays may employ peptides having all or a portion of the amino-acid sequence found in the neo-epitopes formed by enzymatic digestion of IgA1. The peptides may be synthesized chemically or biochemically by methods well known in the art. The peptide may be a cloned IgA1 fragment, Fc or Fab, for example.

Bacteria which secrete the enzyme IgAP may be detected by the invention. Among the bacteria which may be so detected are certain species of Neisseria, Haemophilus and Streptococcus.

Bacteria which secrete IgAP may be distinguished by the method of the present invention. In this method, the bacteria to be distinguished are contacted with IgA1 under conditions suitable for reaction between IgAP and IgA1 to form fragments having neo-epitopes characteristic of bacterial source of IgAP, and the fragments are contacted with antibodies capable of binding specifically with the neo-epitopes. Thus Neisseria gonorrhea may be distinguished from Neisseria meningitidis by contacting the fragments of IgA1 produced by these bacteria with antibody capable of reacting specifically with neo-epitopes produced by reaction of IgA1 with IgAP from Neisseria meningitidis or with antibody capable of reacting specifically with neo-epitopes produced by reaction of IgA1 with IgAP and other enzymes from Neisseria gonorrhea. Bonding between antibodies and fragments is observed by means of labels, for example, or by means of a read-out device, latex agglutinations beads, for example or other means known in the art.

The method of the present invention is especially useful in the diagnosis of gonorrhea. Pathogenic strains of Neisseria gonorrhea, the causative agent of gonorrhea, secrete IgAP. It has been observed that IgA1 from uroginital secretions, vaginal wall and urethra, which have been exposed to Neisseria gonorrhea, and thus to IgAP, is cleaved in vivo by the enzyme. The fragments so produced may be assayed by the method of the present invention. Preferably, the diagnosis comprises contacting a sample, extract from urogenital tract, for example, with one or more antibodies capable of reacting specifically with neo-epitopes on fragments of IgA1 produced in vivo by reaction of IgAP and other enzymes from Neisseria gonorrhea with IgAl from the membrane. The neo-epitopes which form during hydrolysis of IgA1 with IgAP may be further modified in vivo by reaction with other enzymes, hydrolases, for example, from Neisseria gonorrhea. The diagnosis of the present invention involves antibodies which bind specifically to the neo-epitopes on these fragments of IgA1 formed in vivo. These neo-epitopes are characteristic of Neisseria gonorrhea since other bacteria in the vaginal flora either do not contain IgAP and are therefor incapable of splitting IgA1 to form fragments, or do not contain enzymes which further modify the fragments. Neisseria meningitidis, for example, which occasionally inhabits urogenital membranes, produces IgAP which forms fragments of IgA1 but they may be distinguished immunologically from fragments produced by Neisseria gonorrhea. Neisseria gonorrhea may be detected, for example, on an IgAl-producing membrane in the presence of Neisseria meningitidis on said membrane by immunoassay of the membrane with one or more antibodies capable of reacting specifically with epitope on fragments of IgA1 produced in vivo by reaction of IgAP and other enzymes from Neisseria gonorrhea with IgA1 from the membrane, these antibodies being incapable of reacting with fragments of IgA1 produced by IgAP from Neisseria meningitidis. In other embodiments of the present invention, both Neisseria gonorrhea and Neisseria meningitidis may be detected in a single specimen from an IgAP-producing membrane. In these embodiments, an extract, wash, swab or exudate, is contacted with one set of antibodies capable of reacting specifically with neo-epitopes on fragments of IgA1 produced in vivo by the reaction of IgAP and other enzymes from Neisseria gonorrhea and also with another set of antibodies capable of reacting specifically with antigenic site on Neisseria meningitidis, the polysaccharide capsule, for example, or an intracellular antigen. Each set of antibody may be located at a different locus, on two lanes of a solid support, for example, or on different latex beads or inert strips. In this way the reaction of each set may be observed independently of the other set and the presence of either bacteria or of both may be confirmed.

The method of the present invention is also useful in the diagnosis of other diseases caused by bacteria which secrete IgAP. Neisseria meningitidis, for example, may be detected on IgA1-secreting membranes by immunoassay of extract from the membrane with antibody capable of reacting specifically with neo-epitope on fragments of IgA1 produced in vivo by reaction of IgAP from Neisseria meningitidis with IgA1 from the membrane. Neisseria meningitidis may be found in a biological fluid which does not produce IgA1, spinal fluid, for example, by culturing the fluid with IgA1 and assaying for fragments by the method of the present invention. Similarly Haemophilus influenza may be identified.

In preferred methods of the present invention an extract from a membrane is contacted with one or more antibodies capable of reacting specifically with neoepitopes on the fragments under conditions suitable for binding between the antibodies and the fragments, and the binding of antibodies is observed. The antibodies may, for example, be labeled with a group capable of being observed, a radioactive group, enzyme, fluorescent group, chemiluminescent group, antigenic group or other group well known in the art. Other known read-out methods, latex agglutination beads or immunogenic "dip-sticks" employing dry chemistry and membrane technology may likewise be employed to observe the reaction between antibodies and fragments. In certain embodiments, the extract or other sample is contacted first with a substance capable of binding specifically to the Fc portion of IgA1 to "fish" fragments and intact IgA1 from the sample. Preferably this substance is immobilized on a solid support. The solid support is then contacted with sample and antibodies capable of reacting specifically with epitopes on fragments of IgA1 produced by gonorrheal IgAP under conditions suitable for binding between fragments, antibodies and immobilized substance to form a "sandwich" which is observed by methods well known in the art. In other preferred embodiments of the invention, the sample, extract from membrane, for example, is contacted with antibody capable of binding specifically with neo-epitope on the fragment, and is then contacted with peptide having the amino acid sequence of the neo-epitope being determined. Preferably, the peptide is immobilized on a solid support and the antibody is labeled with a group capable of being observed. Peptide, antibody and fragments are allowed to react and label on the solid surface or in solution is observed.

The present invention also sets forth reagents for assay of fragments of IgA1 resulting from the enzymatic digestion of IgA1 and IgAP comprising antibody capable of reacting specifically with neo-epitopes on the fragments but incapable of reacting with intact IgA1. Preferably the reagent is a monoclonal antibody produced by immunization of a host animal with peptide comprising all or a portion off the amino acid sequence of the neoepitopes. The peptide may be, for example, the Fc, Fab or a combination of Fc and Fab fragments resulting from the enzymatic digestion of IgA1 and IgAP. The peptide may also be synthesized chemically or biochemically. The reagents of the present invention may be used to detect IgA1, IgAP and bacteria which secrete IgAP.

The reagent of the present invention for the diagnosis of gonorrhea comprises antibody capable of reacting specifically with fragments of IgA1 produced in vivo by the reaction of enzymes, IgAP and hydrolases, for example, from Neisseria gonorrhea with IgA1 from IgAl-secreting membranes. In preferred embodiments of the invention, the reagent comprises one or more antibodies capable of reacting specifically with neo-epitopes on fragments of IgA1 produced by the reaction of IgA1 with IgAP from Neisseria gonorrhea. In other embodiments, the reagent comprises one or more antibodies capable of reacting specifically with neo-epitope produced by the reaction of fragments of IgA1 resulting from the reaction of IgA1 and IgAP with hydrolases from Neisseria gonorrhea. The reagent for diagnosis of gonorrhea may comprise as well peptide having amino acid sequence of all or a portion of neo-epitope on fragments of IgA1 produced in vivo by the enzymatic digestion of IgA1 by enzymes from Neisseria gonorrhea.

In preferred embodiments, the peptide is immobilized on a solid support.

Kits for diagnosis of gonorrhea are provided comprising one or more antibodies capable of reacting specifically with neo-epitopes on fragments of IgA1 produced in vivo by the reaction of IgAP and other enzymes from Neisseria gonorrhea, and reagents suitable for causing reaction between the antibody or antibodies and the fragments of IgA1 so produced. Kits are also provided for diagnosis of meningitis comprising one or more antibodies capable of capable of reacting specifically with neo-epitopes on fragments of IgA1 produced in vivo by the reaction of IgA1 with IgAP from Neisseria meningitidis, and reagents suitable for causing reaction between the antibodies and the fragments. Kits are also provided for diagnosis of both meningitis and gonorrhea in a single sample from an individual comprising one or more antibodies capable of reacting specifically with neo-epitope on fragments of IgA1 produced by the reaction of IgA1 with IgAP from Neisseria gonorrhea, one or more antibodies capable of reacting specifically with antigenic components of Neisseria meningitidis, and reagents suitable for causing reaction between the antibodies, the fragments and the antigens.

In preferred embodiments of the kits, antibodies are labeled with groups capable of being observed, enzymes, chromophoric groups, fluorescent groups, chemiluminescent groups, radioactive elements, metals or antigenic groups, for example. In these embodiments the kit may comprise in addition reagents for causing the label to be observed. In preferred embodiments of the invention kits comprise substance capable of binding specifically to IgA. Most preferably the substance is antibody capable of binding to Fc portion of IgA1, and is immobilized on a solid support. In other embodiments of the invention, the kits comprise means for observing the reaction between the antibody or antibodies and the fragments of IgA1. Preferred means comprise read-out devices well known in the art, latex agglutination beads, for example, or dip-sticks. The kits may comprise in addition, means for collection of sample from an individual. In other preferred embodiments of the invention wherein reaction between antibody and fragments is observed by enzyme inhibition immunoassay, kits may comprise in addition peptide having amino acid sequence comprising all or a portion of the amino acid sequence of the neo-epitopes on the fragments being determined. Preferably, the peptide is immobilized on a solid support in the kit.

In the method of the present invention, fragments of IgA1 are determined by immunoassay with antibody, preferably monoclonal antibody, which reacts specifically with the fragments of IgA1 produced when the enzyme IgAP reacts with and cleaves intact IgA1. IgA1 has the structure given in the following diagram 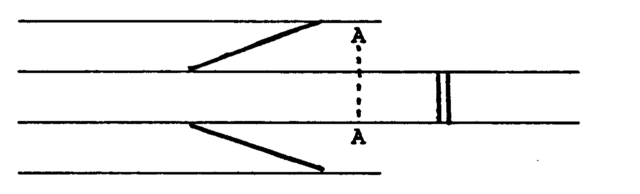
IgAP cleaves IgA1 at the region indicated by the line A-A in the diagram. The precise location of the cleavage site by IgAP differs with bacterial source of the enzyme. Thus IgAP from Neisseria gonorrhea cleaves a threonylprolyl bond at the hinge region (residue 235-236), IgAP from Neisseria meningitidis-1 cleaves IgA1 at pro-ser, (residue 237-238) and IgAP from Haemophilus influenza cleaves IgA1 at pro-ser bond, (residue 231-232). A meningitidis-2 serotype is reported to elaborate an IgAP which cleaves IgA1 at the same pro-thr bond in the hinge which is cleaved by IgAP from Neisseria gonorrhea (Kornfeld, S. et al., (1981) Rev. of Infectious Diseases, 3: 521-534). Over 12 other kinds of IgAP having IgA1 as substrate but with differing cleavage sites have been reported. (Plaut, A. (1983) Annual Review ofMicrobiology, 38: 603-622).

The fragments produced by action of IgAP on IgA1 are generally Fc and Fab fragments, but the neo-epitope comprising amino acids and surrounding carbohydrates exposed by cleavage will differ depending on which bacterial IgAP is used as hydrolytic agent. Since each bacterial IgAP cleaves the IgA1 amino acid chain between a different pair of amino acids, the two fragments which result will have certain terminal sequence of amino acids which will form new epitopes ("neo-epitope") characteristic of the particular IgAP which cleaved the IgA1. Each neo-epitope may be recognized immunologically by a highly specific antibody, a monoclonal antibody, for example, capable of binding to only one particular neo-epitope.

Moreover, because the neo-epitopes presented by the fragments are not available on intact IgA1, the specific antibodies which react with the neo-epitopes do not react with intact IgA1. Because of this specificity the antibodies may also be used to distinguish fragments from intact IgA1 and able to detect fragments in the presence of intact IgA1.

Immunoassay of the present invention for determining fragments comprises competitive binding assays such as enzyme-linked immunoassay or radioimmunoassay, or may be sandwich assays, enzyme inhibition immunoassay or any other immunoassay well known in the art commonly used to observe reaction between antibodies and antigens. Reactions may be observed by means of read-out methods known to the art. Latex agglutination beads, for example, or immunogenic strips employing dry chemistry and membranes having selective pores may be employed. The assays may also be adapted to large scale automated read-out devices for screening large numbers of samples.

In preferred embodiments of the present invention, extract from an IgA1-producing membrane is contacted with one or more antibodies capable of reacting specifically with neo-epitopes on fragments being assayed. Antibodies preferably are labeled with a group capable of being observed. In preferred embodiments, antibodies may also be immobilized on a solid surface. Conditions are maintained for binding between fragments and antibodies. Ionic salts and buffers may, for example, be added to enhance reaction. The bound antibodies are removed from sample and label is measured either on the bound antibody or in the sample.

In other preferred embodiments, the sample is first contacted with a substance capable of binding specifically to the Fc or Fab portion of IgA1. Suitable substances comprise an antibody, preferably a monoclonal antibody, and the IgA binding protein reported by G. Russell-Jones et al. (Russell-Jones, G., Gotschlich, E. and Blake, M. (1984) J. Esp. Med., 160: 1467-1475). In these embodiments, the substance binds to all IgA1 in the sample, including intact IgA1. Preferably the substance is immobilized on a solid surface and IgA1 and IgA1 fragments bind to this solid surface. The solid surface is then contacted with one or more antibodies capable of binding specifically to neo-epitopes on the fragments and this binding is observed.

Other preferred embodiments comprise enzyme-inhibition immunoassay wherein the sample, extract from IgAP-producing membrane, for example, is contacted with one or more antibodies capable of reacting specifically with neo-epitope on the fragment being assayed and is also contacted with peptide having the amino acid sequence of the neo-epitope. The peptide may be immobilized. In embodiments where the peptide is immobilized, for example, the specific antibody reacting with neo-epitopes would be first reacted with samples and then with immobilized peptides. Samples containing neo-epitope would react with antibody and as a result there would be a lack of report of immobilized peptide with antibody.

Generally, the method comprises contacting a sample containing fragments of IgA1 produced during hydrolytic cleavage by IgAP, with antibody capable of reacting with the neo-epitopes on the fragments, these epitopes not being present on intact IgA1. Conditions are provided to cause reaction between fragments and antibodies. Ionic salts, for example and buffers may be added to the solution of fragments. In preferred embodiments of the invention, the antibody is labeled with a group which can be observed, a chromophoric substrate, an enzyme, for example, or a fluorescent group, a radioactive group, a metal, a chemiluminescent group or a suitable antigenic group or hapten. In these embodiments, the reaction between antibody and fragment is observed by measuring this label.

In certain embodiments of the present invention, the immunoassay comprises enzyme inhibition immunoassay (EIIA) with peptide having amino acid sequence of the neo-epitope. The peptide may be synthesized chemically or biochemically. It may, for example, be a cloned IgA1 fragment, Fc or Fab. In these embodiments, sample containing fragments is contacted with antibody capable of reacting specifically with neo-epitope on the fragments and with the peptide. Preferably the peptide is immobilized on a solid surface. Alternately the peptide is labeled with a group capable of being observed and the antibody is immobilized on a solid surface. In either embodiment, the antibody is allowed to react with neo-epitope, peptide is added, and label is observed on solid surface or in sample. Other variations of EIIA well-known in the art may likewise be employed (Engvall, et al., (1980) Methods in Enzymology,Biochemistry and Molecular Biology, Burdon and van Knippenberg, Ed., pp. 350-358).

In other preferred embodiments of the invention, a sample containing fragments of IgA1 is contacted first with a substance capable of reacting specifically with the Fc portion of IgA1. The substance may be, for example, an antibody, most preferably a monoclonal antibody capable of binding specifically to the Fc portion of IgA1. Alternately the binding substance may be the IgA binding protein reported by Russell-Jones et al. Most preferably, this substance is immobilized on a solid support, paper, for example, or on a well in a plate. In these embodiments, the sample is first contacted with the immobilized substance which binds the Fc portion of both the fragment and also any unreacted, intact IgA1. The surface is then contacted with antibody capable of reacting only with neo-epitope on the hydrolyzed fragment of IgA1. The antibody binds only to the fragment, not to the intact IgA1 and is observed by suitable means.

Immunoassay of the present invention for fragments of IgA1 produced by reaction with IgAP is useful in assay of IgA1, of IgAP, in detecting and differentiating between species which produce IgAP and in diagnosis of diseases caused by these bacteria.

The method of the present invention is especially useful in determining bacteria which produce IgAP. A sample containing the bacteria is contacted with substrate IgA1 and then assayed for fragments produced by the IgAP with the IgA1. The bacteria may be cultured prior to measurement in order to increase the sensitivity of the assay. Alternatively the bacteria may be determined in a biological sample from an individual. Pathogenic bacteria which may be measured comprise Neisseria and Haemophilae. The assay is especially useful for diagnosis of diseases caused by the bacteria.

The method of the present invention is especially useful in distinguishing between bacteria in a sample when more than one kind of bacteria which secrete IgAP may be present. In these embodiments, fragments produced by action of IgAP on IgA1 are assayed with antibody which reacts specifically with neo-epitope on the fragments. Monoclonal antibodies, for example, are highly specific reagents capable of reacting with the fragments produced by IgAP from one bacteria but not with the fragments produced by IgAP from the other bacteria. The cleavage site on IgA1 for each bacterial IgAP is known and antibodies may be prepared which are specific for neo-epitopes produced at each cleavage site. In other embodiments where IgA1 is present in the biological sample and reacts with IgAP in vivo, extracts from membranes are assayed for fragments of IgA1 without requiring any intermediate addition of IgA1 and in vitro reaction with IgAP.

The method of the present invention is especially useful in the primary diagnosis of gonorrhea. Certain membranes, urogenital membranes, for example, normally secrete IgA1. It has been observed that in the presence of Neisseria gonorrhea this IgA1 is broken down into fragments. Vaginal swabs, for example, and urethral extract from infected individuals exhibit these fragments. Since the level of secretion of IgA1 rises upon infection by Neisseria gonorrhea (Mulks, M. et al., J. Infect. Dis. (1984) 150: 734-44), the amount of IgA1 present is significant and capable of being observed by the method of the present invention. It appears that IgAP is secreted by Neisseria gonorrhea and reacts in vivo with IgA1 to produce the fragments. In addition, other enzymes from Neisseria gonorrhea may also modify these fragments. Hydrolases, for example, cleave terminal proline from the terminus of the IgA1 fragment (Chen, Kirk et al., (1980) J. Biol. Chem., 255: 1704-1710). These enzymes, however, are incapable of attacking intact IgA1 so both IgAP and other enzymes from pathogenic Neisseria gonorrhea act in vivo to form fragments having neo-epitopes characteristic of gonorrheal infection. The method of the present invention comprises immunoassay of these fragments produced in vivo with highly specific antibodies.

Occasionally Neisseria meningitidis co-exists with Neisseria gonorrhea on the IgA1-producing membranes which results in in vivo production of meningeal IgAP and fragments of IgA1 characteristic of this enzyme as well as fragments produced by gonorrheal IgAP. Preferred embodiments of the present invention provide a method for distinguishing these fragments and thus provide a highly specific assay for gonorrhea. In these embodiments monoclonal antibodies which are capable of reacting specifically with fragments produced by IgAP and other enzymes from Neisseria gonorrhea but are incapable of reacting with fragments produced by Neisseria meningitidis are used for immunoassay. Alternatively, in embodiments employing antibodies incapable of distinguishing between fragments, a separate assay for meningitis may be employed. Immunoassay of a meningeal antigen, a surface antigen or intracellular component, for example, may be used to confirm separately the presence of Neisseria meningitidis.

The method of the present invention may also be to detect Neisseria meningitidis. The bacteria may be detected, for example, on an IgAP-producing membrane by immunoassay with antibodies capable of reacting specifically with fragments of IgA1 produced in vivo on the membrane by the reaction between meningeal IgAP and IgA1.

In other embodiments of the invention, both Neisseria gonorrhea and Neisseria meningitidis may be detected in a single sample from an IgA1-producing membrane. In these embodiments, an extract from the membrane, a wash or swab from vagina or cervix, or a sample of urethral discharge, for example, is contacted with antibodies capable of reacting specifically with neo-epitope on fragments of IgA1 produced in vivo by the reaction of IgAP and other enzymes from Neisseria gonorrhea and also with antibody capable of reacting specifically with antigenic sites, polysaccharide capsule or internal antigens, for example, on Neisseria meningitidis. The immunoassay is conducted in a way which permits separation of reactions. Antibodies may, for example, be immobilized on separate lanes of a solid surface. In this way the reaction with meningeal fragments may be observed separately from reaction with gonorrheal fragments and reaction of both or either may be noted.

The diagnostic method of the present invention comprises generally contacting an extract from an IgA1-producing membrane, vaginal or cervical wash or swab in the case of females or urethral extract in the case of males, with one or more antibodies, preferably monoclonal antibodies, capable of reacting specifically with neo-epitope on fragments of IgA1 produced in vivo by reaction of IgAP and other enzymes from Neisseria gonorrhea with IgA1 from the membrane. The antibodies are caused to react with the fragments and the reaction is observed by any of the methods known to the art. Antibodies may, for example be labeled with a group capable of being observed. In these embodiments, bound antibody is then removed from solution and label is measured either on bound antibody or in solution. In other embodiments, the extract is contacted with a substance capable of binding specifically with the Fc portion of IgA1. The substance is preferably immobilized on a solid support and to separate IgA1, both intact and fragments, from solution. The surface is then contacted with antibodies which bind to neo-epitopes on immobilized fragments but not to intact IgA1. Bound antibodies are then observed on the solid surface. Surface substances comprise antibody, preferably monoclonal antibody, and the IgA binding protein reported by Russell-Jones et al. Other preferred embodiment comprise enzyme inhibition immunoassay (EIIA) in which sample containing fragments is contacted antibodies capable of binding specifically to the fragments and with peptide capable of binding to those antibodies which do not react with sample analyte. Any of the EIIA methods well known in the art may be employed, but generally the method involves peptide, preferably immobilized on a solid surface. The peptide comprises amino acids having the sequence of the neo-epitope of the IgA1 fragments produced in vivo by enzymes from Neisseria gonorrhea.

The present invention also provides reagent when used in the method of the invention comprising one or more monoclonal antibodies capable of reacting with neo-epitope on the fragments. These neo-epitopes are not present on intact IgA1 and the antibodies are incapable of reacting with intact IgA1, that is, with epitopes on the fragments common to IgA1 and the fragments. Preferably, the reagent is monoclonal antibody produced by immunization of a host animal, a BLAB/C mouse, for example, with fragments, Fc or Fab or a combination of Fc and Fab fragments produced by the reaction of IgAP on IgA1. Alternatively, the monoclonal antibodies may be produced by immunization of the host animal with peptide having the amino acid sequence of the neo-epitope being assayed. The peptide may be synthesized chemically or biochemically, by cloning of fragments, for example. Monoclonal antibodies thus produced may be screened against IgA1 and the fragments. Those monoclonal antibodies which react with the fragments but not the intact IgA1 may be used as reagent in the present invention.

The reagent may be used to detect IgA1, IgAP and bacteria which produce IgAP in the immunoassay of the present invention. In preferred embodiments, the reagent comprises antibodies capable of reacting specifically with neo-epitopes on fragments of IgA1 produced in vivo by enzymes, IgAP and hydrolases, from Neisseria gonorrhea. In these embodiments, the reagent may comprise antibodies to the neo-epitope on the Fab fragment, Fc fragment, produced by reaction of gonorrheal IgAP with IgA1 and may in addition comprise, for greater specificity, antibodies to epitopes produced in vivo by other hydrolases, proline iminopeptidase and aminopeptidase-P, for example, from Neisseria gonorrhea. Reagent for diagnosis may comprise, in addition, peptide having the amino acid sequence of neo-epitopes on fragments of IgA1 and of epitopes produced by reaction of gonorrheal hydrolases with these neo-epitopes. In preferred embodiments of the invention, the peptide is immobilized on a solid support.

Reagent for diagnosis of meningitis comprising one or more antibodies capable of reacting specifically with neo-epitope produced by the reaction between meningeal IgAP and IgA1 is also set forth in the present invention. Neisseria meningitidis may be detected on IgA1-producing membranes by immunoassay with this reagent.

The present invention sets forth a peptide reagent having the amino acid sequence of all or a portion of the said neo-epitope, when used in the method of claim 1. The peptide reagent is preferably immobilized on a solid support.

The present invention sets forth also a kit comprising the monoclonal antibody reagent and reagents suitable for causing reaction between the antibody and said IgA1 fragments, when used in a method of the invention. The kit may further contain the peptide reagent comprising the sequence of the neo-epitope to be detected.

The present invention also sets forth kits for diagnosis of gonorrhea in an individual comprising one or more antibodies capable of reacting specifically with neo-epitope on fragments of IgA1 produced in vivo by the reaction of IgA1 with IgAP and other enzymes from Neisseria gonorrhea and reagents for causing reaction between the antibody or antibodies and the fragments of IgA1. Reagents may comprise, for example, ionic salts and buffers suitable for causing the reaction. In preferred embodiments of the invention, the antibodies are labeled with groups capable of being observed, an enzyme, fluorescent group, chemiluminescent group, chromophoric group, metal, antigen, or hapten, for example and may be used in the immunoassay methods generally in use, ELIZA, EIIA, or sandwich immunoassays, for example, or in automated systems employing electronic devices for large scale measurements. The antibodies may also be immobilized on a solid support for use in colorimetric and similar immunoassays and with other read-out methods such as agglutinations or dip-sticks employing wet or dry chemistry. The kits may also comprise reagents for causing label on antibodies to be observed, substrates for labelling enzymes, for example. In preferred embodiments of the invention, the kits comprise in addition substance capable of binding specifically to IgA, most specifically to the Fc portion of IgA1. Preferably this substance is immobilized on a solid support. Embodiments of kits useful in EIIA comprise in addition peptide having amino acid sequence of all or a portion of the amino acid sequence of the neo-epitope being assayed. Preferably this peptide is immobilized on a solid support. Kits may comprise, in addition, means for observing the reaction between the antibodies and the fragments of IgA1. Other embodiments comprise means for collection of sample, swab or pedicle, for example, from an individual to be diagnosed.

The following examples are intended to illustrate the present invention but are not intended to limit the scope of the invention thereby.

### Example 1

This example illustrates the primary diagnosis of gonorrhea in male or female patients.

### a) Sample Preparation

The infected area, vagina in the case of females and ureter in the case of males, is swabbed with an absorbent sterile dacron swab. The swab is immersed in 0.5 ml of a solution containing Tween 20 and a buffer at pH 7.2. The contents of the swab are repeatedly expressed unto the buffer container and removed.

### b) Preparation of Specific Monoclonal Antibody

Monoclonal antibody is prepared by the Kohler and Millstein technique by immunizing a BALB/c mouse with fragments of IgA1 produced by enzymatic digestion with IgAP from Neisseria gonorrhea. Hybridomas are formed by fusion of splenocytes from the mouse with SP-1 cells. Hybridoma cells are screened for those which produce monoclonal antibodies which bind to the fragment but do not bind to intact IgA1.

Selected monoclonal antibodies are linked to gold spheres by methods known in the art.

### c) Separation of IgA1 and Fragments from Sample

Monoclonal antibody for the Fc portion of IgA1 is immobilized on nitrocellulose paper by methods known in the art. A strip of paper is immersed in the sample and left to incubate for 15 minutes. The inert strip is removed from sample and rinsed with phosphate buffered saline (PBS).

### d) Detection of Fragment

The inert strip is placed in solution containing reagent from step b) along with salts and buffers to cause reaction between fragments on the strip and reagent in solution. The strip is observed for color. Black strip indicates binding of labeled monoclonal antibody and is a positive indicator for gonorrhea. A control strip from non-infected sample does not bind monoclonal antibody and remains white.

## Claims

1. Method for detection of IgA1, IgAP and/or bacteria secreting IgAP in a sample, wherein fragments of IgA1 formed in a first step in vivo by enzymatic digestion of IgA1 by IgAP are subjected to immunoassay of said fragments with monoclonal antibody capable of reacting specifically with neo-epitope produced by said enzymatic digestion, but incapable of reacting with intact IgAl.

2. Method of claim 1 wherein said immunoassay comprises radioimmunoassay, enzyme-linked immunoassay or sandwich immunoassay with said antibody or immuno-inhibition enzyme assay with peptide comprising all or a portion of the amino acid sequence characteristic of said neo-epitope.

3. Method according to any claim 1 or claim 2 in which the fragments with said neo-epitope are the products of the further breakdown of the direct product of the digestion of IgAl by IgAP.

4. Method according to any preceding claim in which the sample comprises blood, fraction of blood, extract of membrane which secretes IgAl or other bodily fluid containing IgAl, IgAP and/or bacteria secreting IgAP.

5. Method according to claim 4 in which the bacteria are from the genus Neisseria, Haemophilus or Streptococcus.

6. Method according to any preceding claim for distinguishing between two bacteria, both of which secrete IgAP, in which said antibody reacts with the neo-epitope of fragments from the enzymatic digestion of IgAl by the IgAP of one only of the bacteria, the method optionally including a method for detecting the other bacteria, preferably by detecting the fragments of the enzymatic digestion of IgAl with the IgAP of that other bacteria by a method of any preceding claim using an antibody capable of reacting specifically with neo-epitope on those fragments, or by detecting other specific antigenic sites on said other bacteria by immunoassay.

7. Method according to any preceding claim in which gonorrrhea and/or meningitis is diagnosed.

8. Method according to any preceding claim in which the antibody is labelled and the method includes observing the label.

9. Method according to claim 8 in which the immunoassay is selected from:
(a) a process in which a sample containing IgAl and said IgAl fragments is contacted with a substance, which binds both IgAl and said fragments and which is preferably immobilised on a solid support, and the mixture is then contacted with said labelled antibody and antibody bound to the fragments is observed, and
(b) a process in which a sample containing said IgAl fragments is contacted with said labelled antibody and with a peptide, which has the amino acid sequence of said neoepitope and which is preferably immobilised on a solid support, and antibody bound to peptide is observed.

10. Method according to claim 9(a) in which the substance is capable of binding specifically to the F_{c} or F_{ab} portion of IgAl and is preferably a monoclonal antibody or the IgA binding protein.

11. Use of monoclonal antibody reagent in a method according to any preceding claim said reagent comprising monoclonal antibody capable of reacting specifically with neo-epitope on said fragments but incapable of reacting with intact IgAl.

12. Use of peptide reagent in a method as defined in any of claims 2 to 10, said reagent comprising peptide having the amino acid sequence of all or a portion of said neoepitope.

13. Use according to claim 12 in which the peptide is immobilised on a solid support.

14. Kit for use in a method according to any of claims 1 to 10 comprising a reagent as defined in claim 11 and reagents suitable for causing reaction between said antibody and said fragments of IgAl, optionally including a reagent as defined in claim 12 or 13.

## Patentansprüche

1. Verfahren zur Bestimmung von IgA1, IgAP und/oder IgAP absondernden Bakterien in einer Probe, worin IgA1-Fragmente, die in einem ersten Schritt durch in vivo erfolgenden enzymatischen Abbau von IgA1 durch IgAP gebildet sind, dem Immunoassay der genannten Fragmente mit monoklonalem Antikörper unterzogen werden, der spezifisch mit durch den genannten enzymatischen Abbau erzeugtem Neo-Epitop, aber nicht mit intaktem IgA1 zu reagieren vermag.

2. Verfahren nach Anspruch 1, worin der genannte Immunoassay umfaßt Radioimmunoassay, Enzym-gebundenen Immunoassay oder Sandwich-Immunoassay mit dem genannten Antikörper oder Immuno-Inhibitions-Enzym-Assay mit Peptid, das die gesamte oder einen Teil der für den genannten Neo-Epitop charakteristischen Aminosäure-Sequenz aufweist.

3. Verfahren nach Anspruch 1 oder 2, bei dem die Fragmente mit dem genannten Neo-Epitop die Produkte des weiteren Abbaus des unmittelbaren Produkts des Abbaus von IgA1 durch IgAP sind.

4. Verfahren nach einem der vorhergehenden Ansprüche, bei dem die Probe Blut, Blutfraktion, Extrakt einer IgA1 absondernden Membran oder eine sonstige, IgA1, IgAP und/oder IgAP absondernde Bakterien enthaltende Körperflüssigkeit umfaßt.

5. Verfahren nach Anspruch 4, bei dem es sich um Bakterien der Gattung Neisseria, Haemophilus oder Streptococcus handelt.

6. Verfahren nach einem der vorhergehenden Ansprüche zum Unterscheiden zwischen zwei Bakterien, die beide IgAP absondern, bei welchem der genannte Antikörper mit dem Neo-Epitop von Fragmenten von dem enzymatischen Abbau von IgA1 durch IgAP von nur einem der Bakterien reagiert, wobei das Verfahren wahlweise ein Verfahren zum Bestimmen des anderen Bakteriums umfaßt, vorzugsweise durch Ermittlung der Fragmente des enzymatischen Abbaus von IgA1 durch das IgAP jenes anderen Bakteriums durch ein Verfahren nach einem der vorhergehenden Ansprüche unter Anwendung eines Antikörpers, der spezifisch mit Neo-Epitop auf jenen Fragmenten reagiert oder durch Ermittlung anderer spezifischer Antigenstellen an dem genannten anderen Bakterium durch Immunoassay.

7. Verfahren nach einem der vorhergenden Ansprüche, bei dem Gonorrhoe und/oder Meningitis diagnostiziert werden.

8. Verfahren nach einem der vorhergehenden Ansprüche, bei welchem der Antikörper markiert ist und das Verfahren das Beobachten der Markierung einschließt.

9. Verfahren nach Anspruch 8, bei dem der Immunoassay gewählt wird aus:
(a) einem Verfahren, bei dem eine IgA1 und die genannten IgA1-Fragmente enthaltende Probe in Kontakt gebracht wird mit einer Substanz, die sowohl IgA1 als auch die genannten Fragmente bindet und die vorzugsweise auf einem festen Träger immobilisiert ist, und bei dem das Gemisch anschließend in Kontakt gebracht wird mit dem genannten markierten Antikörper und an die Fragmente gebundener Antikörper beobachtet wird, und
(b) einem Verfahren, bei dem eine die genannten IgA1-fragmente enthaltende Probe in Kontakt gebracht wird mit dem genannten markierten Antikörper und mit einem Peptid, das die Aminosäuresequenz des genannten Neo-Epitops aufweist und vorzugsweise auf einem festen Träger immobilisiert ist, und bei dem an Peptid gebundener Antikörper beobachtet wird.

10. Verfahren nach Anspruch 9 (a), bei dem die Substanz spezifisch an den Fc- oder Fab-Teil von IgA1 zu binden vermag und vorzugsweise einen monoklonalen Antikörper oder das IgA-bindende Protein darstellt.

11. Verwendung von monoklonalem Antikörper-Reagenz in einem Verfahren nach einem der vorhergehenden Ansprüche, welches Reagenz monoklonalen Antikörper enthält, der spezifisch mit Neo-Epitop an den genannten Fragmenten zu reagieren vermag, aber nicht mit intaktem IgA1.

12. Verwendung von Peptid-Reagenz in einem Verfahren nach einem der Ansprüche 2 bis 10, welches Reagenz Peptid umfaßt, das die Aminosäuresequenz des gesamten oder eines Teils des genannten Neo-Epitops aufweist.

13. Verwendung von Anspruch 12, bei der das Peptid auf einem festen Träger immobilisiert ist.

14. Kit zur Anwendung bei einem Verfahren nach einem der Ansprüche 1 bis 10, enthaltend ein Reagenz nach Anspruch 11 sowie Reagenzien, die geeignet sind, eine Reaktion zwischen dem genannten Antikörper und den genannten IgA1-Fragmenten herbeizuführen, vorzugsweise ein Reagenz nach Anspruch 12 oder 13 enthaltend.

## Revendications

1. Méthode de détection de l'IgA1, de l'IgAP et/ou de bactéries secrétant de l'IgaP dans un échantillon, dans laquelle des fragments d'IgA1 formés dans une première étape in vivo par digestion enzymatique de l'IgA1 par l'IgAP sont soumis à un essai immunologique de ces fragments avec un anticorps monoclonal capable de réagir spécifiquement avec le néo-épitope produit par cette digestion enzymatique, mais incapable de réagir avec l'IgA1 intacte.

2. Méthode selon la revendication 1, dans laquelle cet essai immunologique comprend un essai radioimmunologique, un essai immunologique sur enzyme liée ou un essai immunologique sandwich avec cet anticorps ou un essai d'enzyme par immuno-inhibition avec un peptide comprenant tout ou partie de la séquence d'aminoacides caractéristique de ce néo-épitope.

3. Méthode selon l'une quelconque des revendications 1 ou 2, dans laquelle les fragments avec ce néo-épitope sont les produits de la rupture ultérieure du produit direct de la digestion de l'IgA1 par l'IgAP.

4. Méthode selon l'une quelconque des revendications précédentes, dans laquelle l'échantillon comprend du sang, un fragment de sang, un extrait de membrane qui secrète l'IgA1 ou un autro fluide corporel contenant de l'IgA1, de l'IgAP et/ou des bactéries secrétant l'IgAP.

5. Méthode selon la revendication 4, dans laquelle les bactéries appartiennent aux genres Neisseria, Haemophilus ou Streptococcus.

6. Méthode selon l'une quelconque des revendications précédentes pour distinguer entre deux bactéries qui secrètent toutes deux de l'IgAP, dans laquelle cet anticorps réagit avec le néo-épitope de fragments de la digestion enzymatique de l'IgA1 par l'IgAP de l'une seulement des bactéries, la méthode comprenant, si on le désire, une méthode pour détecter les autres bactéries, de préférence en détectant les fragments de la digestion enzymatique de l'IgA1 par l'IgAP de ces autres bactéries par une méthode, selon l'une quelconque des revendications précédentes, utilisant un anticorps capable de réagir spécifiquement avec le néo-épitope sur ces fragments ou en détectant d'autres sites antigénique spécifiques sur ces autres bactéries par un essai immunologique.

7. Méthode selon l'une quelconque des revendications précédentes, dans laquelle on diagnostique la gonorrhée et/ou la méningité.

8. Méthode selon l'une quelconque des revendications précédentes, dans laquelle l'anticorps est marqué et la méthode comprend l'observation du marquage.

9. Méthode selon la revendication 8, dans laquelle l'essai immunologique est choisi parmi :
(a) un procédé dans lequel un échantillon contenant de l'IgA1 et ces fragments d'IgA1 est mis en contact avec une substance qui lie à la fois Iga1 et ces fragments et qui est, de préférence, immobilisée sur un support solide, et la mélange est ensuite mis en contact avec cet anticorps marqué et on observe l'anticorps lié aux fragments, et
(b) un procédé dans lequel un échantillon contenant des fragments d'IgA1 est mis en contact avec cet anticorps marqué et avec un peptide, qui possède la séquence d'aminoacides de ce néo-épitope et qui est, de préférence, immobilisé sur un support solide, et on observe l'anticorps lié au péptide.

10. Méthode selon la revendication 9(a), dans laquelle la substance est capable de se lier spécifiquement à la partie F_{c}ou F_{ab} de l'IgA1 et est, de préférence, un anticorps monoclonal ou la protéine liant l'IgA.

11. Utilisation d'un anticorps monoclonal comme réactif dans une méthode selon l'une quelconque des revendications précédentes, ce réactif comprenant un anticorps monoclonal capable de réagir spécifiquement avec le néo-épitope sur ces fragments, mais incapable de réagir avec l'IgA1 intacte.

12. Utilisation d'un peptide comme réactif dans une méthode telle que définie dans l'une quelconque des revendications 2 à 10, ce réactif comprenant un peptide ayant la séquence d'aminoacides de la totalité ou d'une partie de ce néo-épitope.

13. Utilisation selon la revendication 12, dans laquelle le peptide est immobilisé sur un support solide.

14. Trousse destinée à l'utilisation dans une méthode selon l'une quelconque des revendications 1 à 10. comprenant un réactif tel que défini dans la revendication 11 et des réactifs permettant de provoquer une réaction entre cet anticorps et ces fragments d'IgA1, contenant, si on le désire, un réactif tel que défini dans la revendication 12 ou 13.
